# EUROPÄISCHE PATENTSCHRIFT

(11) **EP 3 278 106 B1**
(45) Veröffentlichungstag und Bekanntmachung des Hinweises auf die Patenterteilung: **07.10.2020**
(21) Anmeldenummer: 16729757.1
(22) Anmeldetag: 24.03.2016
(51) Int. Cl.: A61M 1/02, G01N 21/53, G01N 21/85, G01N 33/49, G01N 21/31, G01N 21/59, G01N 21/84

(54) **VERFAHREN UND VORRICHTUNG ZUR SEPARATION UND ANALYSE VON BLUTBESTANDTEILEN**
METHOD AND DEVICE FOR SEPARATING AND ANALYZING BLOOD CONSTITUENTS
PROCÉDÉ ET DISPOSITIF POUR SÉPARER ET ANALYSER DES COMPOSANTS SANGUINS

(30) Priorität: 02.04.2015 DE 102015004409
(43) Veröffentlichungstag der Anmeldung: 07.02.2018
(73) Patentinhaber: LMB Technologie GmbH, 85445 Schwaig (DE)
(72) Erfinder: NETZ, Uwe, 10717 Berlin (DE); CAPPIUS, Hans-Joachim, 12157 Berlin (DE)
(74) Vertreter: Winter, Brandl, Fürniss, Hübner, Röss, Kaiser, Polte - Partnerschaft mbB
(86) Internationale Anmeldenummer: PCT/DE2016/100145
(87) Internationale Veröffentlichungsnummer: WO 2016/155706

(56) Entgegenhaltungen:
- DE-A1- 19 530 969
- DE-A1-102008 061 695
- DE-A1-102011 008 460
- US-A- 4 227 814
- US-A- 4 807 676
- US-A- 4 969 882
- US-A- 5 958 250
- US-B1- 6 294 094

## Beschreibung

Die Erfindung betrifft ein Verfahren zur Separation und Analyse von Blutbestandteilen gemäß Anspruch 1 sowie eine Vorrichtung zur Separation und Analyse von Blutbestandteilen gemäß Anspruch 13.

Es ist lange bekannter Stand der Technik, Blut zur weiteren Verwendung in seine Bestandteile zu trennen. Eine bekannte Vorrichtung zu Trennung von flüssigen Gemischen, insbesondere Blut, in deren Bestandteile mit Hilfe von Zentrifugieren ist beispielhaft in der DE2308485 A1 beschrieben.

Insbesondere Blut ist, unter anderem aufgrund geringem Spendenaufkommens, eine knappe und wertvolle Ressource und nicht nur deshalb sind medizinisch hohe Qualitätsanforderungen bei der Herstellung bzw. Gewinnung von Blutkonserven, als auch bei deren qualitativen Überwachung im Lagerungsprozess der Blutkonserven zu erfüllen.

Zurzeit ist keine Methode zur nicht-invasiven Qualitätskontrolle von Blut, bzw. insbesondere von Erythrozytenkonzentraten verfügbar. Bisher wird die Qualität in erster Linie durch die Einhaltung von hohen Qualitätsstandards bei der Blutverarbeitung, bei der Ausstattung und bei der fachlichen Schulung des Personals gewährleistet. Verfahren zur Qualitätskontrolle, wie sie heutzutage hauptsächlich verwendet werden, beruhen zumeist entweder auf invasiven Tests, die zu einer Zerstörung von wenigstens 1% der Gesamtmenge führen oder auf der äußeren visuellen Kontrolle durch das Personal. Es ist bekannt, dass das freie Hämoglobin ein Maß für den Zustand der roten Blutkörperchen und daher ein wichtiger Parameter für die Beurteilung der Qualität und der Verwendbarkeit der Erythrozyten Konzentrate vor der Transfusion ist. Insbesondere der Anstieg bzw. die Veränderung der Konzentration des freien Hämoglobins durch den Zerfall der roten Blutkörperchen (Hämolyse) kann heutzutage lediglich durch die visuelle Kontrolle der Blutkonserven erfolgen. Auf Grundlage dieser visuellen Kontrolle, die naturgemäß mit einer Unsicherheit behaftet ist, werden Blutkonserven bei dem Verdacht, dass der Hämolysegrad zu hoch ist nicht mehr verwendet.

Aus anderen Bereichen der Blutverarbeitung sind durchaus Verfahren bzw. Vorrichtungen bekannt, die Verfahren zur Qualitätskontrolle beinhalten, die über zerstörerische Tests bzw. die bloße visuelle Kontrolle hinausgehen. So ist aus der DE10315484A1 ein Verfahren und eine Vorrichtung zur automatischen Filtration von Substanzen bekannt, welches insbesondere auch zur Leukozythendepletion von Blut geeignet ist. Zur Qualitätskontrolle des hergestellten Filtrats ist hier mit Hilfe einer Gewichtsmesseinheit eine Überwachung der Menge und der Qualität des hergestellten Filtrats möglich.

Die DE102011008460A1 beschreibt ein Verfahren zur Herstellung eines leukozytendepletierten Filtrats. Ein Blutpräparat wird aus einem Versorgungsbehälter anhand der Schwerkraft über eine Zuleitung durch einen Leukozytenfilter und nachfolgend in einen Auffangbehälter geleitet. Ein optischer Sensor misst die Hämoglobinkonzentration nach dem Leukozytenfilter und ein Wiegesystem misst das Gewicht des Auffangbehälters. Anhand des Gewichts und der Hämoglobinkonzentration ermittelt eine Recheneinheit das Gesamthämoglobin in dem Auffangbehälter.

Ein optisches Sensormesssystem, das geeignet ist, um mit Hilfe der Messung mit mehreren Lichtwellenlängen Konzentrationen von Stoffen in inhomogenen Gemischen zu bestimmen, ist in der DE102008081695A1 beschrieben.

Die US4969882 A beschreibt ein Verfahren zur Separation von Blutbestandteilen. Rote Blutzellen werden in einem ersten Behälter zentrifugiert und dann über ein Verbindungsstück in einen zweiten Behälter geleitet. Das Gewicht der oberen Phase in dem ersten Behälter wird anhand des Hämatokritwerts und des Gesamtgewichts der nicht separierten roten Blutzellen bestimmt.

Weitere Verfahren und Vorrichtungen zum Verarbeiten von Blut sind darüber hinaus aus US5958250A, US6294094B1, DE19530969A1, US4227814A und US4807676A bekannt.

Es ist Aufgabe der Erfindung, einen oder mehrere Nachteile im Stand der Technik zu vermeiden oder zu vermindern. Es ist insbesondere Aufgabe der Erfindung ein verbessertes Verfahren zur Separation von Blut in dessen Blutbestandteile zu ermöglichen, das es insbesondere erlaubt, in einfacher und genauer Weise, die Bestandteile, z.B. zum Zwecke der Qualitätsüberwachung, während des Verfahrens zu analysieren.

Es ist weiter insbesondere Aufgabe der Erfindung, eine Vorrichtung zu schaffen, mit der ein Verfahren zur Separation von Blut in dessen Blutbestandteile durchgeführt werden kann, das es insbesondere erlaubt in einfacher und genauer Weise, die Bestandteile, z.B. zum Zwecke der Qualitätsüberwachung, während des Verfahrens zu analysieren.

Es ist insbesondere Ziel der Erfindung ein Verfahren zu schaffen, das eine nichtinvasive Qualitätskontrolle von Blutbestandteilen, insbesondere von Erythrozytenkonzentraten, mit Hilfe der Messung bzw. Überwachung des Hämoglobin- und/oder Lipidgehaltes erlaubt.

Die Aufgabe der Erfindung wird hinsichtlich des Verfahrens mit den Merkmalen des unabhängigen Anspruchs 1 und hinsichtlich der Vorrichtung mit den Merkmalen des Anspruchs 13 gelöst.

Das erfindungsgemäße Verfahren und die erfindungsgemäße Vorrichtung können durch weitere Ausgestaltungen, wie sie im Folgenden beschrieben sind, in vorteilhafter Weise ergänzt sein. Diese vorteilhaften Ausgestaltungen sind hinsichtlich des Verfahrens Gegenstand der abhängigen Ansprüche 2-12 und hinsichtlich der Vorrichtung Gegenstand der abhängigen Ansprüche 14-15.

Das erfindungsgemäße Verfahren umfasst die Merkmale des Anspruchs 1.

Dieses Verfahren hat den Vorteil, dass in situ zum Separationsprozess der einen Phase von der zweiten Phase Hämoglobin- und/oder Lipidgehalt in der in das zweite Behältnis gepumpten Phase bestimmbar ist. Aufwendige Analysen im Nachgang zur Separation, die ggfs. noch mit einer Substanz-Entnahme verbunden sind, sind nicht mehr nötig. Ein weiterer Vorteil liegt darin begründet, dass die Analyse ohne eine Entnahme von Substanz möglich ist. Dadurch muss das abgeschlossene System, das die Behältnisse mit den Verbindungsstücken bilden, nicht geöffnet oder verletzt werden. Durch die Analyse der Kennwerte des Hämoglobin- und Lipidgehalts können zudem den Blutbestandteilen weitere Charakteristika zugeordnet werden, die eine qualitative Unterscheidung der Blutbestandteile zulassen.

Es kann bevorzugt vorgesehen sein, dass die Aufnahme der optischen Messwerte zur Bestimmung des Hämoglobin- und/oder des Lipidgehalts gestoppt wird, sobald die vorgesehene Menge der von dem ersten Behältnis in das zweite Behältnis gepumpten Phase aus dem ersten Behältnis in das zweite Behältnis gepumpt wurde. Da die Messungen vorzugweise am Verbindungsstück durch eine optische Transmissionsmessung durchgeführt werden, ist nach der Beendigung des Pumpvorganges keine weitere Messung nötig.

In einer weiteren bevorzugten Ausgestaltung werden die Messwerte zur Bestimmung der Menge des von dem ersten Behältnis in das zweite Behältnis gepumpten Phase und die Messwerte zur Bestimmung des Hämoglobin- und/oder des Lipidgehaltes in einer Recheneinheit weiterverarbeitet. Die Weiterverarbeitung der Messwerte in einer integrierten Auswerteeinheit bietet den Vorteil, dass die Kennwerte zu Hämoglobin- und Lipidgehalt direkt ermittelt und der jeweiligen Konserve mit Blutbestandteilen zugeordnet werden können.

In einer weiteren bevorzugten Ausgestaltung werden die ermittelten Kennwerte dem jeweiligen Behältnis mit den separierten Phasen zuordenbar in einer Datenbank gespeichert und gemeinsam mit einer Kennung des Behältnisses mit den separierten Blutbestandteilen auf einer Ausgabeeinheit ausgegeben. Die ermittelten Kennwerte umfassen den Hämoglobingehalt und/oder den Lipidgehalt und werden aus der Hämoglobin- bzw. Lipidkonzentration und der in das zweite Behältnis gepumpten Menge an Blutbestandteilen bestimmt. Die Konzentration kann bezogen auf ein Volumen oder bezogen auf das Gewicht ermittelt werden. Durch die Anzeige der ermittelten Kennwerte werden dem Anwender in vorteilhafter Weise direkt nach Beendigung des Verfahrens Informationen über die Qualität der gerade getrennten Phase mitgeteilt.

In einer weiteren bevorzugten Ausgestaltung können die aus den Messwerten ermittelten Kennwerte gemeinsam mit einer Kennung des Behältnisses mit den separierten Phasen auf Etiketten ausgegeben werden, wobei die Etiketten geeignet sind, an dem Behältnis mit den separierten Phasen befestigt zu werden. Um zu gewährleisten, dass die Qualitätsangaben zu den in das zweite Behältnis gepumpten Blutbestandteilen auch bei der Weiterverwendung der Blutbestandteile stets mit den Blutbestandteilen selbst verfügbar sind, werden alle diese Blutbestandteile charakterisierenden Informationen auf ein Etikett gedruckt, das dann am Behältnis selbst befestigt werden kann.

In einer weiteren besonders bevorzugten Ausgestaltung ist das Verbindungsstück in seiner Lage fixierbar, wobei sich die Lage des fixierten Verbindungsstückes im Strahlengang einer optischen Messvorrichtung zwischen Strahlenquelle und Detektor befindet. In einer weiteren bevorzugten Ausgestaltung wird die ordnungsgemäße Fixierung des Verbindungsstücks durch einen Sensor überwacht. In einer weiteren besonders bevorzugten Ausgestaltung wird bei nicht ordnungsgemäßer Fixierung des Verbindungsstücks eine Fehlermeldung ausgegeben. In einer weiteren bevorzugten Ausgestaltung ist der Start des Pumpvorganges und/oder Messvorganges nur startbar, wenn das Verbindungsstück ordnungsgemäß fixiert ist. Dies hat den Vorteil, dass vor Beginn des Verfahrens sichergestellt wird, dass sich das Verbindungsstück nahe dem Messort in einer ordnungsgemäßen Position befindet. Bei einer Dejustage des Verbindungsstücks könnten sonst fehlerhafte Messwerte ermittelt werden.

In einer weiteren besonders bevorzugten Ausgestaltung umfasst die Aufnahme der optischen Messwerte die folgenden Schritte
- Bestrahlung der Phasen mit Licht aus einer oder mehreren Lichtquellen mit einem schmalbandigen Wellenlängenbereich, wobei die Blutbestandteile während der Aufnahme der optischen Messwerte durch das Verbindungsstück hindurchfließen
- Detektion des Lichts nach Transmission durch Blutbestandteile in einem Detektor.

In einer weiteren besonders bevorzugten Ausgestaltung werden aus dem im Detektor detektierten Licht die optischen Messwerte für die wellenlängenspezifische Lichtschwächung erfasst. Dabei ist es ganz besonders bevorzugt, dass aus den Messwerten der Lichtschwächung aufgrund der Absorption der Blutbestandteile in der durch das Verbindungsstück hindurchgeleiteten Phase und den Messwerten zur Bestimmung einer Durchflussmenge durch das Verbindungsstück der Gehalt des Hämoglobins in der durch das Verbindungsstück hindurchgeleiteten Phase bestimmt wird. Gleichermaßen ist es ganz besonders bevorzugt, dass aus den Messwerten der Lichtschwächung aufgrund der Streuung der Blutbestandteile in der durch das Verbindungsstück hindurchgeleiteten Phase und den Messwerten zur Bestimmung einer Durchflussmenge durch das Verbindungsstück der Lipid-Gehalt in der durch das Verbindungsstück hindurchgeleiteten Phase bestimmt wird. Durch die kontaktlose Messung in Transmission ist eine Messgenauigkeit gewährleistet. Zudem wird das abgeschlossene System nicht für eine Probenentnahme geöffnet. Das vermindert die Kontaminationsgefahr erheblich.

In einer weiteren bevorzugten Ausgestaltung werden die optischen Messwerte und die Messwerte zur Bestimmung der Durchflussmenge durch das Verbindungsstück mehrfach erfasst. Dabei kann es besonders bevorzugt sein, dass der Hämoglobin- und/oder der Lipid-Gehalt in der Menge, die durch das Verbindungsstück durchgeleitet wurde, durch Integration oder Addition der Messwerte ermittelt wird. Im Falle von Inhomogenitäten in der Verteilung von Lipid- oder Hämoglobingehalt in der in das zweite Behältnis zu pumpenden Phase kann durch eine einzelne Messung keine Aussage über den Gesamtgehalt an Hämoglobin und/oder Lipid getroffen werden. Daher ist es vorteilhaft diese Messungen jeweils bezogen auf ein Teilvolumen der in das zweite Behältnis zu pumpenden Phase durchzuführen oder mehrere zeitlich hintereinander liegende Messungen durchzuführen. Über ein Additions- oder Integrationsverfahren können dann die Gesamtgehalte an Hämoglobin und Lipid ermittelt werden.

In einer weiteren bevorzugten Ausgestaltung wird die durch das Verbindungsstück hindurchgeleitete Phase mit Licht aus mindestens zwei Lichtquellen durchstrahlt, wobei das Licht der mindestens zwei Lichtquellen vom Hämoglobin unterschiedlich stark absorbiert wird. Dabei kann es besonders bevorzugt sein, dass zur Ermittlung der Streuung des Lichts nach Durchstrahlung der durch das Verbindungsstück hindurchgeleiteten Phase die Messwerte des Lichts mit derjenigen Wellenlänge benutzt werden, die vom Hämoglobin weniger absorbiert wird. Durch die unterschiedlich starke Absorption durch das Hämoglobin wird in vorteilhafter Weise gewährleistet, dass auch bei unterschiedlichem Hämoglobingehalt eine hohe Messgenauigkeit erreicht werden kann.

Die erfindungsgemäße Vorrichtung umfasst die Merkmale des Anspruchs 13.

Die erfindungsgemäße Vorrichtung birgt den Vorteil in sich, dass zur Analyse der Kennwerte der Blutbestandteile kein weiterer vom Separationsprozess zeitlich und räumlich abgetrennter Verfahrensschritt durchgeführt werden muss. Das hilft Zeit und Kosten zu sparen.

In einer weiteren bevorzugten Ausgestaltung umfasst die optische Sensoreinheit eine optische Einheit und die Auswerteeinheit. Dabei ist es besonders bevorzugt, dass die optische Einheit einen Detektor und eine oder mehrere schmalbandige Lichtquellen umfasst, wobei die Lichtquellen rotationssymmetrisch um die optische Achse des Detektors angeordnet sind. Ganz besonders bevorzugt umfasst die optische Einheit einen Strahlteiler und einen zweiten Detektor und/oder eine Blende zur Einengung des Strahlbereichs der einzelnen Lichtquellen auf überschneidenden Strahlquerschnitt der Lichtquellen und/oder einen Strahlteiler und einen zweiten Detektor zur Referenzmessung und/oder weitere optische Elemente. In einer weiteren bevorzugten Ausgestaltung ist die optische Einheit in unmittelbarer Nähe zur Vorrichtung zur Fixierung angeordnet. In einer weiteren bevorzugten Ausgestaltung ist die optische Einheit geeignet, die Lichtschwächung aufgrund der Streuung und/oder der Absorption von Blutbestandteilen in dem Verbindungsstück zu messen.

In einer weiteren bevorzugten Ausgestaltung ist die Auswerteeinheil mit einer Einrichtung zur Ausgabe und/oder Speicherung der Messwerte und/oder Kennwerte verbunden. Dabei ist es besonders bevorzugt, dass die Einrichtung zur Ausgabe und/oder Speicherung der Messwerte und/oder Kennwerte eine Anzeige und/oder einen Drucker und/oder eine Datenbank umfasst.

Die Erfindung wird nachfolgend anhand mehrerer Ausführungsbeispiele näher erläutert. Es zeigen:
- Figur 1: Verfahren zur Separation von Blutbestandteilen und deren Analyse
- Figur 2: Verfahren zur Separation von Blutbestandteilen und deren Analyse mit Ausgabe der Analysedaten
- Figur 3: Verfahren zur Separation von Blutbestandteilen und deren Analyse mit Überwachung der ordnungsgemäßen Positionierung des Untersuchungsgegenstandes
- Figur 4: Verfahren zur Separation von Blutbestandteilen und deren Analyse mit Überwachung des Prozessablaufs
- Figur 5: Vorrichtung zur Durchführung eines Verfahrens zur Separation und Analyse von Blutbestandteilen
- Figur 6: Optische Sensoreinheit zur Analyse von Blutbestandteilen, Auswertung, Speicherung und Ausgabe der Ergebnisse
- Figur 7: Optische Einheit zur Analyse von Blutbestandteilen

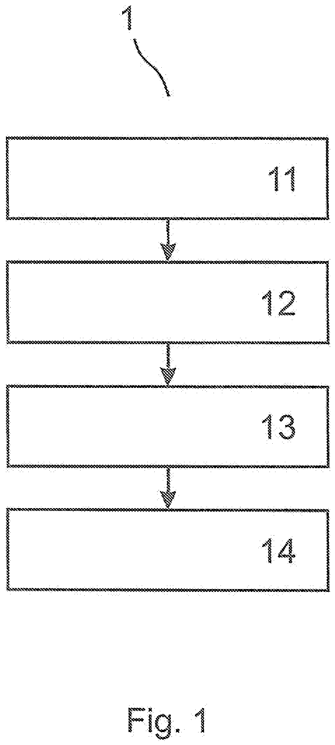

Figur 1 zeigt die Verfahrensschritte des erfindungsgemäßen Verfahrens 1 zur Separation von Blutbestandteilen und Analyse der Blutbestandteile, Bei Verfahren dieser Art werden in Blutkonserven nach der Entnahme bzw. dem Blutspendevorgang die Blutbestandteile in ihrem Behältnis getrennt. Hierzu dient meist ein Zentrifugationsprozess, bei dem das Blut in eine leichte Phase, dem Serum oder Plasma, und in eine schwere Phase, vornehmlich die im Blut enthaltenen Zellen, getrennt wird. Nach dem Zentrifugieren liegen in einem ersten Behältnis 23, dass meist ein Blutbeutel ist, die Blutbestandteile in zwei Phasen vor. Zur Separation dieser beiden Phasen voneinander dient das erfindungsgemäße Verfahren 1, bei dem während des Separationsprozesses die Blutbestandteile in einem Analyseprozess auf ihre Zusammensetzung hin untersucht werden. Dieser Prozess beginnt mit dem Start eines Pumpprozesses 11, mit deren Hilfe eine der Phasen aus einem ersten Behältnis 23, in dem beide Phasen nach dem Zentrifugationsprozess getrennt vorliegen, über ein Verbindungsstück 29 in ein zweites Behältnis 24 gepumpt wird. Das Verbindungsstück 29 verbindet das erste Behältnis 23 mit dem zweiten Behältnis 24. Das erste und zweite Behältnis 23, 24 bilden zusammen mit dem Verbindungsstück 29 ein geschlossenes System. In einer alternativen Ausführungsform kann das geschlossene System weitere Behältnisse 23, 24 und Verbindungsstücke 29 umfassen. Unter Pumpen im Sinne der Erfindung werden alle Maßnahmen verstanden, die dazu dienen, Blutbestandteile von dem ersten Behältnis 23 in das zweite Behältnis 24 zu befördern. Dies kann beispielsweise über die Veränderung der Druckverhältnisse in dem ersten und/oder zweiten Behältnis 23, 24 erfolgen. Die Druckverhältnisse in den Behältnissen 23, 24 können dadurch verändert werden, dass in dem zweiten Behältnis 24 ein Unterdruck erzeugt wird, in dessen Folge die Blutbestandteile in das zweite Behältnis 24 gesogen werden. Weiterhin besteht die Möglichkeit in dem ersten Behältnis 23 einen Überdruck zu erzeugen und so die Blutbestandteile in den zweiten Beutel 24 zu drücken. Dies kann in einfacher Weise dadurch erfolgen, dass das erste Behältnis 23 zusammengedrückt wird. Unmittelbar nach dem Start des Pumpprozesses 11 wird mit der Aufnahme der Messwerte zur Bestimmung der Menge der von dem ersten Behältnis 23 in das zweite Behältnis 24 gepumpten Menge an Blutbestandteilen 12 begonnen. Weiterhin wird nach dem Start des Pumpprozesses 11 die Aufnahme der Messwerte zur Analyse der von dem ersten Blutbehältnis 23 in das zweite Behältnis 24 gepumpten Blutbestandteile 13 begonnen. Sobald die vorgesehene Menge der in das zweite Behältnis 24 zu pumpenden Phase erreicht ist, wird der Pumpprozess beendet 14.

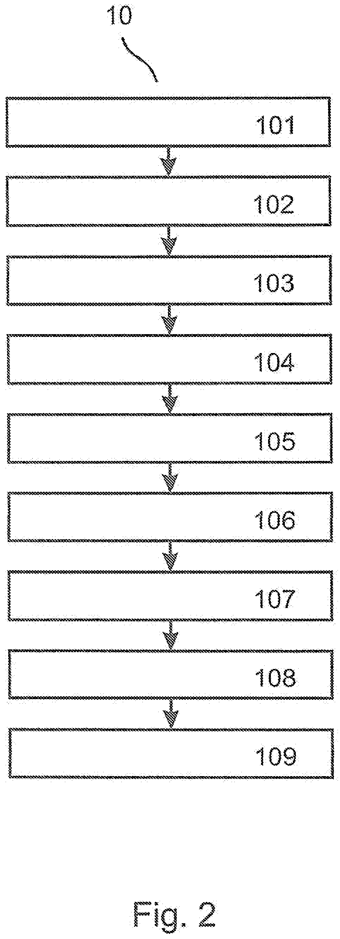

Ein Verfahren zur Separation von Blutbestandteilen und gleichzeitiger Analyse der separierten Phasen 10 ist in Figur 2 dargestellt. Dieses Verfahren 10 baut auf dem in Figur 1 beschriebenen Verfahren 1 auf und ist um weitere Verfahrensschritte 105, 107, 108, 109 ergänzt. Die ergänzten Verfahrensschritte 105, 107, 108, 109 sind jeder für sich einzeln zum in Figur 1 beschriebenen Verfahren 1 ergänzbar. In einem ersten optionalen Verfahrensschritt wird mit Beendigung des Pumpvorganges 14, 104 auch die Aufnahme der Messwerte beendet 105. Trotz der Trennung der Blutbestandteile in zwei Phasen nach dem Zentrifugieren können in den einzelnen Phasen inhomogene Verteilungen der Blutbestandteile wie Hämoglobingehalt oder Lipidgehalt vorliegen. Daher werden die Messwerte zur Bestimmung der Blutbestandteile mehrfach aufgenommen 103. Da insbesondere bei einer inhomogenen Verteilung der Blutbestandteile die mehrfach aufgenommenen Messwerte zur Analyse der Blutbestandteile zur Bestimmung des Gesamtgehalts der Blutbestandteile in der in das zweite Behältnis 24 gepumpten Menge abhängig sind von der zu diesem Zeitpunkt bereits an der optischen Einheit 25 vorbei in das zweite Behältnis 24 gepumpten Menge der abgetrennten Phase werden die in das zweite Behältnis gepumpten Mengen ebenfalls mehrfach gemessen 104. In einer besonders bevorzugten Ausführungsform werden die Messungen der gepumpten Menge 104 und die Messungen zur Analyse der Blutbestandteile 103 so durchgeführt, dass sich jedem Messwert zur Analyse der Blutbestandteile ein Messwert der gepumpten Menge zuordnen lässt. Aus den aufgenommenen Messwerten zur Analyse der Blutbestandteile 103 und zur Bestimmung der in das zweite Behältnis 24 gepumpten Menge 104 der abgetrennten Phase werden in einem weiteren Verfahrensschritt in einer Auswerteeinheit 252 die Kennwerte der in das zweite Behältnis gepumpten Blutbestandteile bestimmt 106.

In einer bevorzugten Ausgestaltung der Erfindung werden die Messungen zur Analyse der Blutbestandteile 103 mehrmals periodisch durchgeführt. Bei einer konstanten Pumpleistung wird dann jede Einzelmessung zur Analyse der Blutbestandteile 103 mit einer in dem jeweiligen Intervall in das zweite Behältnis gepumpten Menge an Blutbeslanteilen korreliert. In einer besonders bevorzugten Ausgestaltung der Erfindung werden die so ermittelten Einzelwerte aufaddiert oder für eine Integration verwendet, um die Gesamtmengen an Hämoglobin und/oder Lipid in der in das zweite Behältnis 24 gepumpten Menge an Blutbestandteilen zu ermitteln. In einer weiteren besonders bevorzugten Ausführungsform werden auch die in das zweite Behältnis 24 gepumpte Menge 102 periodisch bestimmt, wobei die Periodenlänge der Bestimmung der gepumpten Menge 102 gleich der Periodenlänge der Messung zur Analyse der Blutbestandteile 103 ist. Jeden Messwert zur Bestimmung der gepumpten Menge 102 kann so ein Messwert zur Analyse der Blutbestandteile 103 zugeordnet werden. Die Bestimmung der Gesamthämoglobin und/oder Lipidgehalte erfolgt dann wiederum durch Integration oder Summation der Einzelergebnisse. Wird die Menge der in das zweite Behältnis 24 gepumpten Phase 102 an einem anderen Ort bestimmt als die Messungen zur Analyse der Blutbestanteile 103 durchgeführt wird, so kommt es zu einem Fehler bei der Zuordnung der einzelnen Messwerte. Daher wird in einer weiteren bevorzugten Ausgestaltung der Erfindung durch eine entsprechende Korrektur jede Einzelmessung zur Analyse der Blutbestandteile 103 dem Volumenelement oder Mengenelement zugeordnet, an dem die Messung stattgefunden hat.

In einem weiteren optionalen Verfahrensschritt werden die so ermittelten Kennwerte der in das zweite Behältnis 24 gepumpten Blutbestandteile in einer Datenbank gespeichert 107. Die Speicherung kann die ermittelten Messwerte und/oder die berechneten Kennwerte umfassen. Die Messwerte und/oder Kennwerte werden in der Datenbank der Kennung der jeweiligen Blutkonserve zugeordnet. Aus der Datenbank sind die gespeicherten Daten wieder abrufbar. In einer weiteren Ausgestaltung der Erfindung werden die ermittelten Kennwerte auf einer Anzeigeeinrichtung ausgegeben. In einer weiteren bevorzugten-Ausgestaltung der Erfindung werden die Kennwerte zusammen mit der Kennung der Blutkonserve auf einem Etikett ausgedruckt, wobei das Etikett an der Blutkonserve selbst befestigbar ist. Sollten aus vorangegangenen Messungen bereits die Kennwerte der Blutkonserve vor der Separation der Blutbestandteile bekannt sein, so werden in einer weiteren besonders bevorzugten Ausführungsform aus den Kennwerten der Blutkonserve vor der Durchführung des erfindungsgemäßen Verfahrens 1, 10 und den im Verfahren 1, 10 für die in das zweite Behältnis 24 gepumpten Blutbestandteile ermittelten Kennwerte die Kennwerte für die im ersten Behältnis 23 verbleibenden Blutbestandteile ermittelt.

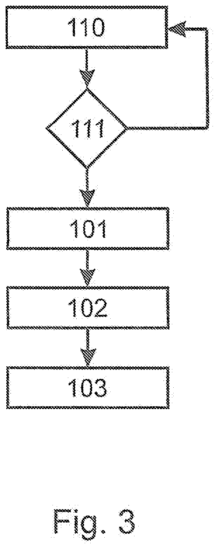

In einem weiteren Ausführungsbeispiel der Erfindung wird die ordnungsgemäße Positionierung des zur Analyse der Blutbestandteile verwendeten Teils des Behältnisses 23, 24 oder des Verbindungsstücks 29 überwacht (Figur 3). In dem vorliegenden Beispiel wird dies durch einen mechanischen Taster 27 realisiert, der ein elektronisches Signal an die Steuereinheit 20 liefert, das zu erkennen gibt, wenn das zur Analyse der Blutbestandteile verwendete Teil des Behältnisses 23, 24 oder des Verbindungsstücks 29 richtig positioniert ist. In einer bevorzugten Ausführungsform lässt sich der Pumpvorgang erst starten, wenn über den Sensor zur Überwachung der korrekten Positionierung ein entsprechendes Signal die Steuereinheit 20 erreicht. Durch diese Steuerung des Pumpvorgangs 111 wird sichergestellt, dass keine fehlerhaften Messwerte aufgrund von Abweichungen in der Anordnung des zur Analyse der Blutbestandteile verwendeten Teils des Behältnisses 23, 24 oder des Verbindungsstücks 29 aufgenommen werden. Die Abfrage über die korrekte Positionierung kann auch kontinuierlich erfolgen. Alternativ kann bei richtiger Positionierung durch den Taster 27 ein Signal zur Freigabe des Prozesses 1, 10 ausgelöst werden.

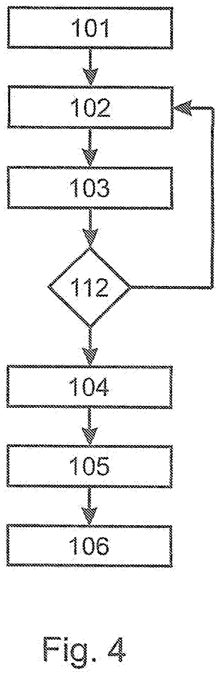

In Figur 4 ist ein Teilverfahren zur Steuerung des Pumpvorganges dargestellt. Nach den Start des Pumpvorganges 11, 101 und der Aufnahme der Messwerte zur Bestimmung der gepumpten Menge 12, 102 und zur Analyse der Blutbestandteile 13, 103 wird überprüft, ob die vorgesehene Menge bereits in das zweite Behältnis 24 gepumpt wurde. Die vorgesehene Menge wird entweder durch einen vorgegebenen Bedarf des Anwenders vorgegeben oder dadurch bestimmt, dass die zuvor per Zentrifugation abgetrennte Phase vollständig in das zweite Behältnis gepumpt wurde. Bei einer vorgegebenen Menge wird die bereits in das zweite Behältnis 24 gepumpte Menge überwacht 112 und der Pumpvorgang beendet 14, 104, sobald die vorgegebene Menge erreicht ist. Soll eine der beispielsweise durch Zentrifugation im ersten Behältnis 23 voneinander getrennten Phasen nahezu vollständig in das zweite Behältnis 24 gepumpt werden, so wird ermittelt, wann der Zeitpunkt erreicht ist, dass die beiden Phasen nahezu vollständig voneinander in den jeweiligen Behältnissen 23, 24 getrennt sind. Die Überwachung kann beispielsweise durch einen optischen Sensor erfolgen, der über die unterschiedlichen optischen Eigenschaften der getrennten Phasen die Position der Grenzfläche zwischen den beiden Phasen ermittelt und ein Signal an die Steuereinheit 20 übermittelt, wenn die beiden Phasen nahezu vollständig auf die zwei Behältnisse aufgeteilt sind. Über die Steuereinheit 20 wird dann der Pumpprozess beendet 14, 104. In einer besonders bevorzugten Ausfübrungsform der Erfindung wird zur Überwachung der Grenzfläche zwischen den voneinander abgetrennten Phasen die Sensoreinheit zur Aufnahme der Messwerte zur Analyse der Blutbestandteile 25, 251 verwendet. Alternativ kann auch ein separater optischer Sensor verwendet werden. Weiterhin ist auch möglich, nach der Zentrifugation die Menge der in das zweite Behältnis 24 zu pumpenden Blutbestandteile zu bestimmen und nach Erreichen der gepumpten Menge den Pumpprozess zu beenden 14, 104.

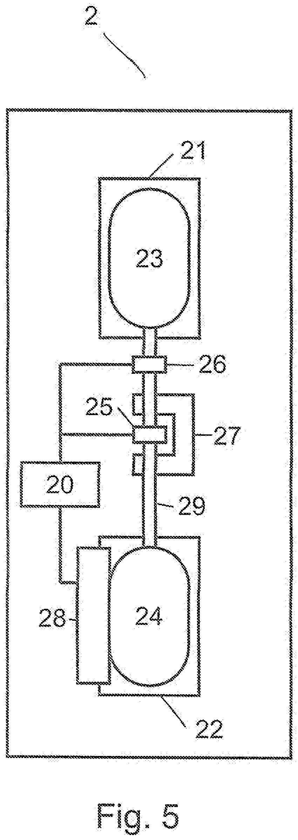

In Figur 5 ist der Aufbau der Vorrichtung zur Separation von Blutbestandteilen und Analyse von Blutbestandteilen 2 dargestellt. Die Vorrichtung 2 umfasst eine erste Vorrichtung 21 zur Aufnahme eines ersten Blutbehältnisses 23 und eine zweite Vorrichtung 22 zur Aufnahme eines zweiten Blutbehältnisses 24, wobei die Blutbehältnisse 23, 24 über ein Verbindungsstück 29 verbunden sind. Weiterhin umfasst die Vorrichtung zur Separation von Blutbestandteilen und Analyse von Blutbestandteilen 2 eine optische Sensoreinheit 25, die dazu geeignet ist, optische Messwerte der in das zweite Behältnis 24 gepumpten Blutbestandteile zu erfassen, sowie eine Vorrichtung 27 zur Fixierung des Verbindungsstücks 29. Weiterhin umfasst die erfindungsgemäße Ausführungsform eine Vorrichtung 28 zum Pumpen von Blutbestandteilen von einem ersten Behältnis 23 in ein zweites Behältnis 24 sowie eine Vorrichtung zum Messen der Menge der vom ersten Behältnis in das zweite Behältnis gepumpten Blutbestandteile 26, 253. Die Vorrichtung zum Pumpen 28, die optische Sensor-Einheit 25 und die Vorrichtung zum Messen der Menge der vom ersten Behältnis in das zweite Behältnis gepumpten Blutbestandteile 26, 253 sind mit einer Steuereinheit 20 verbunden. In einer bevorzugten Ausführungsform ist die optische Einheit 25 in unmittelbarer Nähe der Fixierungsvorrichtung 27 angeordnet.

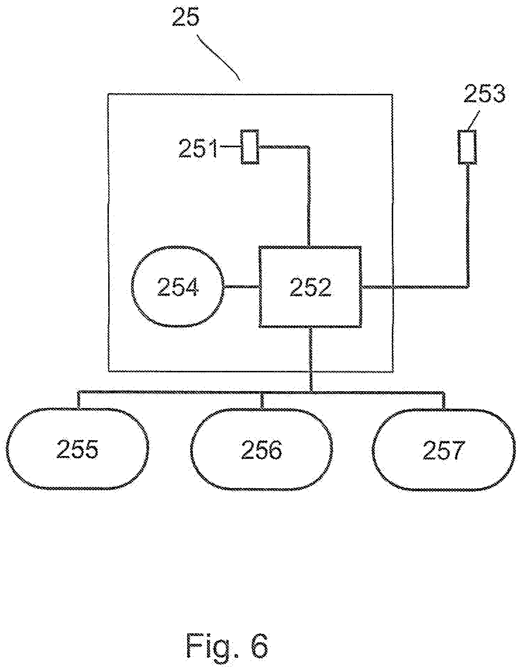

Der Aufbau der optischen Sensor-Einheit 25 ist in Figur 6 dargestellt. Die optische Sensor-Einheit 25 umfasst eine optische Einheit 251 zur Aufnahme der Messwerte zur Analyse von Blutbestandteilen, eine Auswerteeinheit 252 zur Auswertung von erfassten Messdaten und einen Speicher 254 zum Abspeichern von Messdaten und/oder Kennwerten. Die optische Sensoreinheit 25 ist weiterhin verbunden mit einer Vorrichtung zur Bestimmung der von einem ersten Behältnis in ein zweites Behältnis gepumpten Menge von Blutbestandteilen 26, 253. In einer bevorzugten Ausführungsform ist die optische Sensoreinheit 25 verbunden mit einer Anzeigeeinrichtung 255 und einem Drucker 256 zur Ausgabe von bedruckten Etiketten. In einer besonders bevorzugten Ausgestaltung der Erfindung ist die optische Sensoreinheit mit einer Datenbank 257 verbunden, auf die zur Abfrage von den ermittelten Messwerten oder daraus bestimmten Kennwerten von externen Stellen zugegriffen werden kann. In einer weiteren bevorzugten Ausführungsform der Erfindung sind die Steuereinheit 20 und die Auswerteeinheit 252 eine einzelne Einheit.

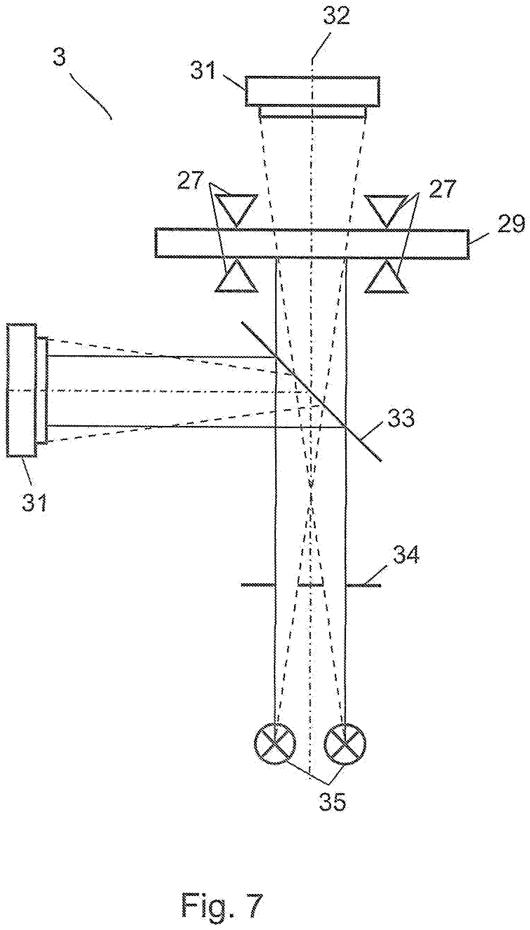

Figur 7 zeigt den Aufbau der optischen Einheit 251. Mehrere monochromatische und/oder schmalbandige Lichtquellen 35 sind rotationssymmetrisch um die optische Achse 32 eines Detektors 31 angeordnet. Die optische Einheit 251 ist hierbei so angeordnet, dass sich der zur Analyse der Blutbestandteile verwendete Teil des Behältnisses 23, 24 oder des Verbindungsstücks 29 im Strahlengang der optischen Einheit 251 befindet und von dem auf den Detektor 31 fallenden Licht durchstrahlt wird. Bevorzugt ist der durchstrahlte Bereich ebenfalls rotationssymmetrisch zur optischen Achse 32, wodurch sich für alle Lichtquellen 35 eine identische optische Weglänge ergibt. In einer bevorzugten Ausführungsform sind die Lichtquellen 35 dicht an der optischen Achse 32 angeordnet. Dies führt zu nur geringen Abweichungen des jeweils vom auf den Detektor 31 fallenden Licht durchstrahlten Probenbereiches. Weiterhin wird in einer bevorzugten Ausführungsform ein breitbandiger Detektor 31 verwendet. In einer besonders bevorzugten Ausführungsform wird eine Blende 34 verwendet, um den Strahlquerschnitt einzuschränken und so dafür Sorge zu tragen, dass der Strahlquerschnitt im Bereich der Probe auf denjenigen Querschnitt beschränkt wird, in dem sich die Strahlquerschnitte aller Lichtquellen 35 überdecken. In einer weiteren besonders bevorzugten Ausgestaltung der Erfindung wird alternativ oder zusätzlich zur Blende 34 eine Linse, die eine möglichst große Menge Licht auf die Probe bündelt, angeordnet In einer weiteren besonders bevorzugten Ausgestaltung der Erfindung wird durch einen Strahlteiler der vor der Probe angeordnet ist, ein Teilstrahl ausgekoppelt und direkt auf einen zweiten Detektor 31 geleitet. Durch diese Anordnung wird der ausgekoppelte Teilstrahl für eine Referenzmessung verwendet.

### BEZUGSZEICHENLISTE

1, 10 Verfahren zur Separation von Blutbestandteilen und Analyse der Blutbestandteile
11, 101 Start des Pumpvorgangs von Blutbestandteilen von einem Behältnis in ein anderes Behältnis
12, 102 Aufnahme von Messwerten zur Bestimmung der in das zweite Behältnis gepumpten Menge
13, 103 Aufnahme von optischen Messwerten zur Bestimmung des Hämoglobin- und/oder Lipidgehalts
14, 104 Beendigung des Pumpvorganges von Blutbestandteilen
105 Beendigung der Aufnahme der Messwerte
106 Bestimmung von Kennwerten der Blutbestandteile aus den aufgenommenen Messwerten in einer Recheneinheit
107 Speicherung der Kennwerte der Blutbestandteile und der Kennung des Behältnisses in einer Datenbank
108 Ausgabe der Kennwerte der Blutbestandteile in einer Anzeige
109 Ausgabe der Kennwerte der Blutbestandteile auf einem Etikett
110 Überwachung der ordnungsgemäßen Fixierung des Verbindungsstücks zwischen den beiden Behältnissen durch einen Sensor
111 Steuerung des Starts des Pumpvorgangs
112 Steuerung der Beendigung des Pumpvorgangs
2 Vorrichtung zur Separation von Blutbestandteilen und Analyse von Blutbestandteilen
20 Steuereinheit
21 Vorrichtung zur Aufnahme eines ersten Behältnisses
22 Vorrichtung zur Aufnahme eines zweiten Behältnisses
23 erstes Behältnis
24 zweites Behältnis
25 optische Sensor-Einheit
26, 253 Einrichtung zur Bestimmung der in das zweite Behältnis gepumpten Menge
27 Vorrichtung zur Fixierung des Verbindungstücks zwischen dem ersten und dem zweiten Behältnis
28 Vorrichtung zum Pumpen von Blutbestandteilen von einem ersten Behältnis in ein zweites Behältnis
29 Verbindungsstück zwischen einem ersten und einem zweiten Behältnis
251, 3 optische Einheit
252 Steuer- und Auswerteeinheit
254 Speicher
255 Anzeigeeinrichtung
256 Drucker
257 Datenbank
31 Detektor
32 optische Achse
33 Strahlteiler
34 Blende
35 Lichtquelle

## Patentansprüche

1. Verfahren (1, 10) zur Separation von Blutbestandteilen, die in einem ersten Behältnis (23) in eine leichte Phase und eine schwere Phase getrennt vorliegen, und Analyse der Blutbestandteile, umfassend folgende Verfahrensschritte:
Pumpen (11, 101) von einer der beiden Phasen aus dem ersten Behältnis (23) in ein zweites Behältnis (24), wobei das erste Behältnis (23) mit dem zweiten Behältnis (24) über ein Verbindungsstück (29) verbunden ist,
Aufnahme (12, 102) von Messwerten zur Bestimmung der Menge der von dem ersten Behältnis (23) in das zweite Behältnis (24) gepumpten Phase,
Aufnahme (13, 103) von optischen Messwerten zur Bestimmung des Hämoglobin- und/oder des Lipidgehaltes der von dem ersten Behältnis (23) in das zweite Behältnis (24) gepumpten Phase während des Pumpens (11, 101) einer der beiden Phasen aus dem ersten Behältnis (23) in das zweite Behältnis (24),
Ermittlung von Kennwerten der durch das Verbindungsstück (29) gepumpten Phase aus den Messwerten zur Bestimmung der Menge der
aus dem ersten Behältnis (23) in das zweite Behältnis (24) gepumpten Phase und den optischen Messwerten zur Bestimmung des Hämoglobin- und/oder des Lipidgehaltes, wobei die Kennwerte den Hämoglobin- und/oder Lipidgehalt umfassen.

2. Verfahren (1, 10) nach Anspruch 1, **dadurch gekennzeichnet, dass** die Aufnahme der optischen Messwerte zur Bestimmung des Hämoglobin- und/oder des Lipidgehalts gestoppt wird, sobald eine vorgesehene Menge der von dem ersten Behältnis (23) in das zweite Behältnis (24) gepumpten Phase aus dem ersten Behältnis (23) in das zweite Behältnis (24) gepumpt wurde.

3. Verfahren (1, 10) nach Anspruch 1 oder 2, **dadurch gekennzeichnet, dass** die Kennwerte der in das zweite Behältnis (24) gepumpten Phase in einer Datenbank (257) gespeichert werden.

4. Verfahren (1, 10) nach Anspruch 3, **dadurch gekennzeichnet, dass** die Kennwerte gemeinsam mit einer Kennung des zweiten Behältnisses (24) mit der gepumpten Phase auf einer Anzeigeeinrichtung (255, 256) ausgegeben und auf einem Etikett ausgedruckt werden, wobei das Etikett geeignet ist, an dem zweiten Behältnis (24) mit der gepumpten Phase befestigt zu werden.

5. Verfahren (1, 10) nach einem oder mehreren der vorhergehenden Ansprüche 1 bis 4, **dadurch gekennzeichnet, dass** das Verbindungsstück (29) im Strahlengang einer optischen Messvorrichtung (251, 3) zwischen einer Lichtquelle (35) und einem Detektor (31) fixiert ist.

6. Verfahren (1, 10) nach einem oder mehreren der vorhergehenden Ansprüche 1 bis 5, **dadurch gekennzeichnet, dass** eine ordnungsgemäße Fixierung des Verbindungsstücks (29) durch einen Sensor überwacht wird, wobei bei nicht ordnungsgemäßer Fixierung des Verbindungsstücks (29) eine Fehlermeldung ausgegeben wird, und/oder wobei ein Start des Pumpens nur startbar ist, wenn das Verbindungsstück (29) ordnungsgemäß fixiert ist.

7. Verfahren (1, 10) nach einem oder mehreren der vorhergehenden Ansprüche 1 bis 6, **dadurch gekennzeichnet, dass** die Aufnahme der optischen Messwerte folgende Schritte umfasst:
Bestrahlung der gepumpten Phase mit Licht aus einer oder mehreren Lichtquellen (35) mit einem schmalbandigen Wellenlängenbereich, und
Detektion des Lichts nach Transmission durch die in das zweite Behältnis (24) gepumpten Phase in einem Detektor (31).

8. Verfahren (1, 10) nach Anspruch 7, **dadurch gekennzeichnet, dass** aus dem im Detektor (31) detektierten Licht optische Messwerte für die wellenlängenspezifische Lichtschwächung erfasst werden.

9. Verfahren (1, 10) nach Anspruch 8, **dadurch gekennzeichnet, dass** aus den optischen Messwerten der wellenlängenspezifischen Lichtschwächung aufgrund einer Absorption der Blutbestandteile in der durch das Verbindungsstück gepumpten Phase und den Messwerten zur Bestimmung der Menge der gepumpten Phase im zweiten Behältnis (24) der Gehalt des Hämoglobins in der durch das Verbindungsstück gepumpten Phase bestimmt wird.

10. Verfahren (1, 10) nach Anspruch 8 oder 9, **dadurch gekennzeichnet, dass** aus den optischen Messwerten der wellenlängenspezifischen Lichtschwächung aufgrund einer Streuung der Blutbestandteile in der durch das Verbindungsstück gepumpten Phase und den Messwerten zur Bestimmung der Menge der gepumpten Phase im zweiten Behältnis (24) der Lipid-Gehalt in der durch das Verbindungsstück gepumpten Phase bestimmt wird.

11. Verfahren (1, 10) nach einem oder mehreren der vorhergehenden Ansprüche 1 bis 10, **dadurch gekennzeichnet, dass** die optischen Messwerte und die Messwerte zur Bestimmung der Menge der gepumpten Phase mehrfach erfasst werden, wobei insbesondere der Hämoglobin- und/oder Lipid-Gehalt in der gepumpten Phase durch Integration der optischen Messwerte ermittelt wird.

12. Verfahren (1, 10) nach einem oder mehreren der vorhergehenden Ansprüche 1 bis 11, **dadurch gekennzeichnet, dass** die durch das Verbindungsstück (29) gepumpte Phase mit Licht aus mindestens zwei Lichtquellen (35) durchstrahlt wird, wobei ein Licht der mindestens zwei Lichtquellen (35) vom Hämoglobin unterschiedlich stark absorbiert wird, und wobei zur Ermittlung einer Streuung des Lichts nach Durchstrahlung der durch das Verbindungsstück (29) gepumpten Phase Messwerte des Lichts mit derjenigen Wellenlänge benutzt werden, die vom Hämoglobin weniger absorbiert wird.

13. Vorrichtung (2) eingerichtet zur Separation von Blutbestandteilen, die in einem ersten Behältnis (23) in eine leichte Phase und eine schwere Phase getrennt vorliegen, und Analyse der Blutbestandteile nach dem Verfahren nach einem oder mehreren der Ansprüche 1 bis 12, wobei die Vorrichtung (2) folgende Elemente umfasst:
das erste Behältnis (23) und ein zweites Behältnis (24), eine Vorrichtung zur Aufnahme (21) des ersten Behältnisses (23), in der das erste Behältnis (23) aufgenommen ist,
eine Vorrichtung zur Aufnahme (22) des zweiten Behältnisses (24), in der das zweite Behältnis (24) aufgenommen ist,
eine Vorrichtung (28) eingerichtet zum Pumpen von einer der beiden Phasen von dem ersten Behältnis (23) in das zweite Behältnis (24),
ein Verbindungsstück (29),
eine Vorrichtung zur Fixierung (27) des Verbindungsstücks (29) zwischen dem ersten Behältnis (23) und dem zweiten Behältnis (24), in der das Verbindungsstück (29) aufgenommen ist,
eine optische Sensor-Einheit (25), eingerichtet zur Aufnahme von optischen Messwerten zur Bestimmung des Hämoglobin- und/oder Lipidgehaltes der von dem ersten Behältnis (23) in das zweite Behältnis (24) gepumpten Phase während des Pumpens einer der beiden Phasen aus dem ersten Behältnis (23) in das zweite Behältnis (24),
Einrichtung (26, 253) eingerichtet zur Bestimmung der Menge der von dem ersten Behältnis (23) in das zweite Behältnis (24) gepumpten Phase, und
eine Auswerteeinheit (252) eingerichtet zur Bestimmung von Kennwerten der durch das Verbindungsstück (29) gepumpten Phase aus den Messwerten zur Bestimmung der Menge des von dem ersten Behältnis (23) in das zweite Behältnis (24) gepumpten Phase und den optischen Messwerten zur Bestimmung des Hämoglobin- und/oder des Lipidgehaltes, wobei die Kennwerte den Hämoglobin- und/oder Lipidgehalt umfassen.

14. Vorrichtung (2) nach Anspruch 13, **dadurch gekennzeichnet, dass** die optische Sensor-Einheit (25) eine optische Einheit (251) und die Auswerteeinheit (252) umfasst, wobei die optische Einheit (251)
(a) einen Detektor (31) und mehrere schmalbandige Lichtquellen (35) umfasst, wobei die mehreren schmalbandigen Lichtquellen (35) rotationssymmetrisch um eine optische Achse (32) des Detektors (31) angeordnet sind, wodurch sich für alle der mehreren schmalbandigen Lichtquellen (35) eine identische optische Weglänge ergibt und/oder
(b) in unmittelbarer Nähe zur Vorrichtung zur Fixierung (27) angeordnet ist, und/oder
(c) geeignet ist, eine Lichtschwächung aufgrund einer Lichtstreuung und/oder einer Absorption von Blutbestandteilen in dem Verbindungsstück (29) zu messen.

15. Vorrichtung (2) nach Anspruch 13 oder 14, die umfasst:
eine Einrichtung zur Ausgabe und/oder Speicherung der Messwerte und/oder Kennwerte, die mit der Auswerteeinheit (252) verbunden ist, wobei die Einrichtung zur Ausgabe und/oder Speicherung der Messwerte und/oder Kennwerte eine Anzeige (255) und/oder einen Drucker (256) und/oder eine Datenbank (257) umfasst.

## Claims

1. A Method (1, 10) for separation of blood components which are present, separated into a light phase and a heavy phase, in a first container (23), and analysis of the blood components, comprising the following method steps:
pumping (11, 101) of one of the two phases from the first container (23) into a second container (24), the first container (23) being connected to the second container (24) via a connecting piece (29),
recording (12, 102) of measurement values for determination of the quantity of the phase pumped from the first container (23) into the second container (24),
recording (13, 103) of optical measurement values for determination of the haemoglobin and/or lipid content of the phase pumped from the first container (23) into the second container (24) during the pumping (11, 101) of one of the two phases from the first container (23) into the second container (24),
determining of characteristic values of the phase pumped through the connecting piece (29) from the measurement values for determination of the quantity of the phase pumped from the first container (23) into the second container (24) and the optical measurement values for determination of the haemoglobin and/or lipid content, wherein the characteristic values comprise the haemoglobin and/or lipid content.

2. The method (1, 10) according to claim 1, **characterized in that** the recording of the optical measurement values for determination of the haemoglobin and/or lipid content is stopped as soon as a predetermined quantity of the phase pumped from the first container (23) into the second container (24) has been pumped from the first container (23) into the second container (24).

3. The method (1, 10) according to claim 1 or 2, **characterized in that** the characteristic values of the phase pumped into the second container (24) are stored in a database (257).

4. The method (1, 10) according to claim 3, **characterized in that** the characteristic values are, together with an identification of the second container (24) with the pumped phase, output on a display device (255, 256) and printed on a label, the label being adapted to be attached to the second container (24) with the pumped phase.

5. The method (1, 10) according to one or more of the preceding claims 1 to 4, **characterized in that** the connecting piece (29) is fixed in the beam path of an optical measuring device (251, 3) between a light source (35) and a detector (31).

6. The method (1, 10) according to one or more of the preceding claims 1 to 5, **characterized in that** a correct fixing of the connecting piece (29) is monitored by a sensor, wherein an error message is output when the connecting piece (29) is not correctly fixed, and/or wherein a start of the pumping is only startable when the connecting piece (29) is correctly fixed.

7. The method (1, 10) according to one or more of the preceding claims 1 to 6, **characterized in that** the recording of the optical measurement values comprises the following steps:
irradiating the pumped phase with light from one or more light sources (35) having a narrow-band wavelength range, and
detecting the light after transmission through the phase pumped into the second container (24) in a detector (31).

8. The method (1, 10) according to claim 7, **characterized in that** optical measurement values for the wavelength-specific light attenuation are detected from the light detected in the detector (31).

9. The method (1, 10) according to claim 8, **characterized in that** the content of haemoglobin in the phase pumped through the connecting piece is determined from the optical measurement values of the wavelength-specific light attenuation due to an absorption of the blood components in the phase pumped through the connecting piece and the measurement values for determination of the amount of the pumped phase in the second container (24).

10. The method (1, 10) according to claim 8 or 9, **characterized in that** the lipid content in the phase pumped through the connecting piece is determined from the optical measurement values of the wavelength-specific light attenuation due to a scattering of the blood components in the phase pumped through the connecting piece and the measurement values for determination of the amount of the pumped phase in the second container (24).

11. The method (1, 10) according to one or more of the preceding claims 1 to 10, **characterized in that** the optical measurement values and the measurement values for determination of the quantity of the pumped phase are recorded multiple times, wherein in particular the haemoglobin and/or lipid content in the pumped phase is determined by integration of the optical measurement values.

12. The method (1, 10) according to one or more of the preceding claims 1 to 11, **characterized in that** the phase pumped through the connecting piece (29) is irradiated with light from at least two light sources (35), wherein a light from the at least two light sources (35) is absorbed by the haemoglobin to a different extent, and wherein measurement values of the light with the wavelength which is absorbed less by the haemoglobin are used for determination of a scattering of the light after irradiation of the phase pumped through the connecting piece (29).

13. A device (2) adapted for separation of blood components which are present, separated into a light phase and a heavy phase, in a first container (23) and analysis of the blood components according to the method of one or more of the claims 1 to 12, wherein the apparatus (2) comprises the following elements:
the first container (23) and a second container (24),
a device for reception (21) of the first container (23), in which the first container (23) is received,
a device for reception (22) of the second container (24), in which the second container (24) is received,
a device (28) adapted for pumping of one of the two phases from the first container (23) into the second container (24),
a connecting piece (29),
a device for fixing (27) the connecting piece (29) between the first container (23) and the second container (24), in which the connecting piece (29) is received,
an optical sensor unit (25) adapted for reception of optical measurement values for determination of the haemoglobin and/or lipid content of the phase pumped from the first container (23) into the second container (24) during the pumping of one of the two phases from the first container (23) into the second container (24),
unit (26, 253) adapted for determination of the quantity of the phase pumped from the first container (23) into the second container (24), and
an evaluation unit (252) adapted for determination of characteristic values of the phase pumped through the connecting piece (29) from the measurement values for determination of the quantity of the phase pumped from the first container (23) into the second container (24) and the optical measurement values for determination of the haemoglobin and/or lipid content, wherein the characteristic values comprise the haemoglobin and/or lipid content.

14. The device (2) according to claim 13, **characterized in that** the optical sensor unit (25) comprises an optical unit (251) and the evaluation unit (252), wherein the optical unit (251)
(a) comprises a detector (31) and multiple narrow-band light sources (35), the multiple narrow-band light sources (35) being arranged rotationally symmetrically about an optical axis (32) of the detector (31), resulting in an identical optical path length for all of the multiple narrow-band light sources (35), and/or
(b) is arranged in close proximity to the device for fixing (27), and/or
(c) adapted to measure light attenuation due to light scattering and/or an absorption of blood components in the connecting piece (29).

15. The device (2) according to claim 13 or 14, comprising:
a device for outputting and/or storing the measurement values and/or characteristic values, which is connected with the evaluation unit (252), wherein the device for outputting and/or storing the measurement values and/or characteristic values comprises a display (255) and/or a printer (256) and/or a database (257).

## Revendications

1. Procédé (1, 10) pour la séparation de composants sanguins qui sont présents séparés en une phase légère et une phase lourde dans un premier récipient (23), et l'analyse des composants sanguins, comprenant les étapes de procédé suivantes:
pompage (11, 101) de l'une des deux phases du premier récipient (23) dans un deuxième récipient (24), où le premier récipient (23) est relié au deuxième récipient (24) par le biais d'un tronçon de liaison (29),
réception (12, 102) de valeurs mesurées pour déterminer la quantité de la phase pompée du premier récipient (23) dans le deuxième récipient (24),
réception (13, 103) de valeurs mesurées optiques pour déterminer la teneur en hémoglobine et/ou en lipides de la phase pompée du premier récipient (23) dans le deuxième récipient (24) pendant le pompage (11, 101) de l'une des deux phases du premier récipient (23) dans le deuxième récipient (24),
détermination de valeurs caractéristiques de la phase pompée à travers le tronçon de liaison (29) à partir des valeurs mesurées pour déterminer la quantité de la phase pompée du premier récipient (23) dans le deuxième récipient (24) et des valeurs mesurées optiques pour déterminer la teneur en hémoglobine et/ou en lipides, où les valeurs caractéristiques comprennent la teneur en hémoglobine et/ou en lipides.

2. Procédé (1, 10) selon la revendication 1, **caractérisé en ce que** la réception des valeurs mesurées optiques pour déterminer la teneur en hémoglobine et/ou en lipides est arrêtée dès qu'une quantité prévue de la phase pompée du premier récipient (23) dans le deuxième récipient (24) a été pompée du premier récipient (23) dans le deuxième récipient (24).

3. Procédé (1, 10) selon la revendication 1 ou 2, **caractérisé en ce que** les valeurs caractéristiques de la phase pompée dans le deuxième récipient (24) sont stockées dans une base de données (257).

4. Procédé (1, 10) selon la revendication 3, **caractérisé en ce que** les valeurs caractéristiques sont délivrées ainsi qu'un identifiant du deuxième récipient (24) avec la phase pompée sur un dispositif d'affichage (255, 256) et sont imprimées sur une étiquette, où l'étiquette convient pour être fixée sur le deuxième récipient (24) avec la phase pompée.

5. Procédé (1, 10) selon une ou plusieurs des revendications 1 à 4 précédentes, **caractérisé en ce que** le tronçon de liaison (29) est fixé dans le trajet du faisceau d'un dispositif de mesure optique (251, 3) entre une source de lumière (35) et un détecteur (31).

6. Procédé (1, 10) selon une ou plusieurs des revendications 1 à 5 précédentes, **caractérisé en ce qu'**une fixation correcte du tronçon de liaison (29) est surveillée par un capteur, où dans le cas d'une fixation incorrecte du tronçon de liaison (29), un message d'erreur est délivré et/ou où un démarrage du pompage ne peut être démarré que quand le tronçon de liaison (29) est correctement fixé.

7. Procédé (1, 10) selon une ou plusieurs des revendications 1 à 6 précédentes, **caractérisé en ce que** la réception des valeurs mesurées optiques comprend les étapes suivantes:
irradiation de la phase pompée avec de la lumière provenant d'une ou plusieurs sources de lumière (35) avec une plage de longueurs d'onde à bande étroite, et
détection de la lumière après transmission à travers la phase pompée dans le deuxième récipient (24) dans un détecteur (31).

8. Procédé (1, 10) selon la revendication 7, **caractérisé en ce que** des valeurs mesurées optiques pour l'atténuation de la lumière spécifique de la longueur d'onde sont collectées à partir de la lumière détectée dans le détecteur (31).

9. Procédé (1, 10) selon la revendication 8, **caractérisé en ce qu'**à partir des valeurs mesurées optiques de l'atténuation de la lumière spécifique de la longueur d'onde du fait d'une absorption des composants sanguins dans la phase pompée à travers le tronçon de liaison et des valeurs mesurées pour la détermination de la quantité de la phase pompée dans le deuxième récipient (24), la teneur de l'hémoglobine dans la phase pompée à travers le tronçon de liaison est déterminée.

10. Procédé (1, 10) selon la revendication 8 ou 9, **caractérisé en ce qu'**à partir des valeurs mesurées optiques de l'atténuation de la lumière spécifique de la longueur d'onde du fait d'une diffusion des composants sanguins dans la phase pompée à travers le tronçon de liaison et des valeurs mesurées pour la détermination de la quantité de la phase pompée dans le deuxième récipient (24) la teneur en lipides dans la phase pompée à travers le tronçon de liaison est déterminée.

11. Procédé (1, 10) selon une ou plusieurs des revendications 1 à 10 précédentes, **caractérisé en ce que** les valeurs mesurées optiques et les valeurs mesurées pour la détermination de la quantité de la phase pompée sont collectées plusieurs fois, où en particulier la teneur en hémoglobine et/ou en lipides dans la phase pompée est déterminée par intégration des valeurs mesurées optiques.

12. Procédé (1, 10) selon une ou plusieurs des revendications 1 à 11 précédentes, **caractérisé en ce que** la phase pompée à travers le tronçon de liaison (29) est irradiée par de la lumière provenant d'au moins deux sources de lumière (35), où une lumière des au moins deux sources de lumière (35) est absorbée par l'hémoglobine plus ou moins fortement, et où les valeurs mesurées de la lumière avec la longueur d'onde qui est moins absorbée par l'hémoglobine sont utilisées pour déterminer une diffusion de la lumière après irradiation de la phase pompée à travers le tronçon de liaison (29).

13. Dispositif (2) installé pour la séparation de composants sanguins qui sont présents séparés en une phase légère et une phase lourde dans un premier récipient (23), et l'analyse des composants sanguins selon le procédé selon une ou plusieurs des revendications 1 à 12, où le dispositif (2) comprend les éléments suivants:
le premier récipient (23) et un deuxième récipient (24),
un dispositif pour la réception (21) du premier récipient (23) dans lequel le premier récipient (23) est reçu,
un dispositif pour la réception (22) du deuxième récipient (24), dans lequel le deuxième récipient (24) est reçu,
un dispositif (28) installé pour pomper l'une des deux phases du premier récipient (23) dans le deuxième récipient (24),
un tronçon de liaison (29),
un dispositif pour la fixation (27) du tronçon de liaison (29) entre le premier récipient (23) et le deuxième récipient (24), dans lequel le tronçon de liaison (29) est reçu,
une unité de capteur optique (25), installée pour la réception de valeurs mesurées optiques pour la détermination de la teneur en hémoglobine et/ou en lipides de la phase pompée du premier récipient (23) dans le deuxième récipient (24) pendant le pompage de l'une des deux phases du premier récipient (23) dans le deuxième récipient (24),
un dispositif (26, 253) installé pour la détermination de la quantité de la phase pompée du premier récipient (23) dans le deuxième récipient (24),
et
une unité d'évaluation (252) installée pour la détermination de valeurs caractéristiques de la phase pompée à travers le tronçon de liaison (29) à partir des valeurs mesurées pour la détermination de la quantité de la phase pompée du premier récipient (23) dans le deuxième récipient (24) et des valeurs mesurées optiques pour la détermination de la teneur en hémoglobine et/ou en lipides, où les valeurs caractéristiques comprennent la teneur en hémoglobine et/ou en lipides.

14. Dispositif (2) selon la revendication 13, **caractérisé en ce que** l'unité de capteur optique (25) comprend une unité optique (251) et l'unité d'évaluation (252), où l'unité optique (251)
(a) comprend un détecteur (31) et plusieurs sources de lumière à bande étroite (35), où les plusieurs sources de lumière à bande étroite (35) sont disposées de manière symétrique en rotation autour d'un axe optique (32) du détecteur (31), de sorte qu'il en résulte pour toutes les plusieurs sources de lumière à bande étroite (35) une longueur de trajet optique identique et/ou
(b) est disposée à proximité immédiate du dispositif pour la fixation (27) et/ou
(c) est appropriée pour mesurer une atténuation de la lumière du fait d'une diffusion de la lumière et/ou d'une absorption de composants sanguins dans le tronçon de liaison (29).

15. Dispositif (2) selon la revendication 13 ou 14, qui comprend:
un dispositif pour la délivrance et/ou le stockage des valeurs mesurées et/ou des valeurs caractéristiques, qui est relié à l'unité d'évaluation (252), où le dispositif pour la délivrance et/ou le stockage des valeurs mesurées et/ou des valeurs caractéristiques comprend un dispositif d'affichage (255) et/ou une imprimante (256) et/ou une base de données (257).
